# FASCICULE DE BREVET EUROPEEN

(11) **EP 2 291 209 B1**
(45) Date de publication et mention de la délivrance du brevet: **15.07.2015**
(21) Numéro de dépôt: 09766053.4
(22) Date de dépôt: 25.05.2009
(51) Int. Cl.: A61M 1/10

(54) **Appareil permettant d'appliquer une pression pulsatile déterminée sur un dispositif médical**
Vorrichtung zur Anwendung eines pulsierenden Drucks auf ein medizinisches Gerät
Device for applying a pulsatile pressure to a medical device

(30) Priorité: 27.05.2008 FR 0802871
(43) Date de publication de la demande: 09.03.2011
(73) Titulaire: Nour, Sayed, 92370 Chaville (FR); Chastanier, Pierre, 75008 Paris (FR)
(72) Inventeur: NOUR, Sayed, 92370 Chaville (FR)
(74) Mandataire: Thomas, Nadine
(86) Numéro de dépôt international: PCT/FR2009/050965
(87) Numéro de publication internationale: WO 2009/153491

(56) Documents cités:
- EP-A1- 0 972 949
- EP-A2- 1 452 195
- WO-A2-2007/081612
- US-A1- 2001 016 676

## Description

La présente invention a pour objet un appareil permettant d'appliquer une pression pulsatile déterminée sur un dispositif médical.

Dans la description qui suit, l'expression « dispositif médical » doit être comprise comme désignant une tubulure, un cathéter, une combinaison ou tout autre dispositif médical.

Conceptuellement, le système cardio-vasculaire est un circuit hydraulique, fermé, sous pression, tapissé intérieurement par des cellules endothéliales. Le fonctionnement de ces cellules endothéliales est régulé par la pulsation cardiaque qui entraîne des variations de pression dans les vaisseaux et donc un cisaillement de ces cellules, ce qui les stimule. Ces forces tangentielles du stress de cisaillement (shear stress) sont indispensables au maintien de la fonction endothéliale comprenant le tonus vasculaire par la synthèse d'oxyde nitrique (NOS), la coagulation du sang, la réponse inflammatoire, l'immunité, l'athérosclérose, l'angiogénèse et l'apoptose. La fonction endothéliale est très importante puisqu'elle contrôle l'embryogenèse, la morphogenèse, l'organogenèse ainsi que le maintien d'un organisme sain.

Toute intervention sur ce circuit, telle que, par exemple, une pathologie ou une opération chirurgicale, entraîne un dysfonctionnement endothélial avec des conséquences pouvant être dramatiques.

Dans le domaine de l'assistance circulatoire, de nombreux appareils pulsatiles sont actuellement utilisés. Parmi ceux-ci on peut citer, par exemple, les appareils de contre-pulsion extracorporelle externe amélioré (EECP ; abrégé de la nomenclature anglaise External Enhanced CounterPulsation), les appareils d'assistance ventriculaire gauche (LVAD : abrégé de la nomenclature anglaise Left Ventricular Assist Devices), les ballons de contre-pulsion intra aortique (IABP : abrégé de la nomenclature anglaise Intra Aortic Balloon Pump), etc.

Toutefois, les appareils pulsatiles existants actuellement nécessitent, pour créer cette fonction pulsatile, des consoles équipées de systèmes informatiques sophistiqués, d'électrocardiogramme, de moniteurs cardiopulmonaires, d'alarmes, etc. Or toutes ces consoles sont très coûteuses, très volumineuses et nécessitent des personnels soignants bien formés ainsi que des ingénieurs et techniciens pour un fonctionnement correct.

On connaît le document US 2001/016676 qui décrit un système empêchant la condensation dans une pompe intra aortique à ballon.

L'invention se propose donc de simplifier les appareils existants grâce à un nouvel appareil destiné à appliquer une pression pulsatile déterminée sur un dispositif médical.

Ledit appareil d'application d'une pression pulsatile déterminée sur un dispositif médical comprend :
- un moyen de prélèvement adapté à prélever du fluide depuis une source de fluide, en flux continu, sous haute pression ;
- un moyen de transformation adapté à transformer ledit fluide en un fluide en flux pulsatile sous basse pression ;
- au moins un moyen d'application pour appliquer ledit fluide en flux pulsatile sous basse pression sur ledit dispositif médical ; et
- un moyen d'évacuation du fluide.

L'appareil de l'invention comprend, en outre, une enceinte, ladite enceinte étant :
- adaptée à être fixée temporairement à la source de fluide, en flux continu, sous haute pression, pour prélever un volume fini de fluide ;
- fixée au moyen de prélèvement pour permettre le prélèvement du volume fini de fluide qu'elle contient ; et
- fixée au moyen d'évacuation pour créer un circuit fermé de fluide.

Ledit appareil a l'avantage d'être beaucoup plus simple et plus petit en dimension que les consoles existantes. Il est donc moins coûteux en production et en fonctionnement.

L'appareil de l'invention présente l'avantage de créer la pulsatilité appliquée sur le dispositif médical grâce à une simple source de fluide en flux continu sous haute pression. Cette source de fluide peut être celle présente dans toute chambre médicalisée, par exemple les bouteilles de gaz ou de liquide haute pression.

La présence de l'enceinte permet de prélever un volume fini de fluide sous haute pression. Puis, une fois ce volume de fluide prélevé, la fixation entre l'enceinte, donc l'appareil de l'invention, et la source de fluide peut être éliminée. L'appareil de l'invention devient alors un appareil autonome.

Les fixations de l'enceinte au moyen de prélèvement et au moyen d'évacuation peuvent être réalisées à demeure ou non.

Le moyen d'évacuation étant relié à ladite enceinte, l'appareil de l'invention présente un circuit fermé de fluide. Il n'y a donc pas de perte de fluide. L'appareil de l'invention peut fonctionner de manière portative pour autant que le volume prélevé soit suffisant pour alimenter l'ensemble du circuit enceinte-moyen de transformation-moyen d'application-moyen d'évacuation.

Dans un mode de réalisation de l'invention, l'appareil de l'invention comprend, en outre, un corps creux présentant deux extrémités et, de la périphérie vers le centre du corps creux, une paroi externe, une chambre creuse et une membrane flexible définissant un passage traversant reliant les deux extrémités, adapté à l'insertion d'un dispositif médical, ledit moyen d'application traversant ladite paroi externe, aboutissant dans la chambre creuse et étant adapté à appliquer une pression sur la membrane flexible de manière pulsée et le moyen d'évacuation partant de la chambre creuse et traversant également ladite paroi externe.

En fonctionnement, le dispositif médical est inséré dans le passage traversant et le fluide, en flux pulsatile sous basse pression, est appliqué, au travers de la chambre creuse, sur la membrane flexible. La membrane flexible subit un mouvement de compression/décompression qu'elle transmet au dispositif médical. Le dispositif médical est alors un dispositif pulsatile.

Dans un autre mode de réalisation, l'appareil de l'invention comporte, en outre, une enceinte, telle que définie plus haut ainsi qu'un corps creux tel que défini plus haut.

Dans une forme d'exécution de l'appareil de l'invention, ledit moyen de transformation *est* un système de pistons-ressorts et de compartiment.

Cet ensemble piston-ressort associé à un compartiment est un système relativement simple permettant la transformation d'une source continue de fluide haute pression en des pulsations rythmiques de fluide sous basse pression (voir ci-dessous).

Dans une forme d'exécution, ledit moyen de transformation est commandé électromécaniquement.

La présence d'une commande électromécanique permet à un opérateur de commander le fonctionnement des pistons-ressorts et, ainsi, de choisir la fréquence de pulsation ainsi que la pression souhaitées.

Dans une forme d'exécution, l'appareil de l'invention comporte, en outre, des moyens de contrôle et d'alarme. Ces moyens de contrôle et d'alarme permettent, par exemple, de détecter toute fuite de fluide, de mesurer le taux d'oxygène dans le sang du patient ou la fréquence cardiaque du patient et de déclencher une alarme en cas de danger et/ou de stopper les pulsations de l'appareil.

Dans une forme d'exécution particulière, la commande électromécanique et/ou les moyens de contrôle et d'alarme enregistrent un historique des événements en créant, par exemple, une base de données afin de pouvoir être interrogée, plus tard, par un opérateur.

L'invention sera mieux comprise par référence aux dessins annexés, dans lesquels :
- la figure 1 est une représentation schématique d'un premier appareil permettant d'appliquer une pression pulsatile ;
- la figure 2 est une représentation schématique d'un appareil de l'invention, dans lequel l'appareil comprend une enceinte ; et
- la figure 3 est une représentation schématique d'un deuxième appareil comprenant un corps creux.

Dans les dessins, les mêmes chiffres (unité et dizaine) de référence désignent des structures correspondantes d'une figure à l'autre, le chiffre des centaines distinguant les différentes variantes.

Dans les dessins, la circulation du fluide est schématisée par des flèches.

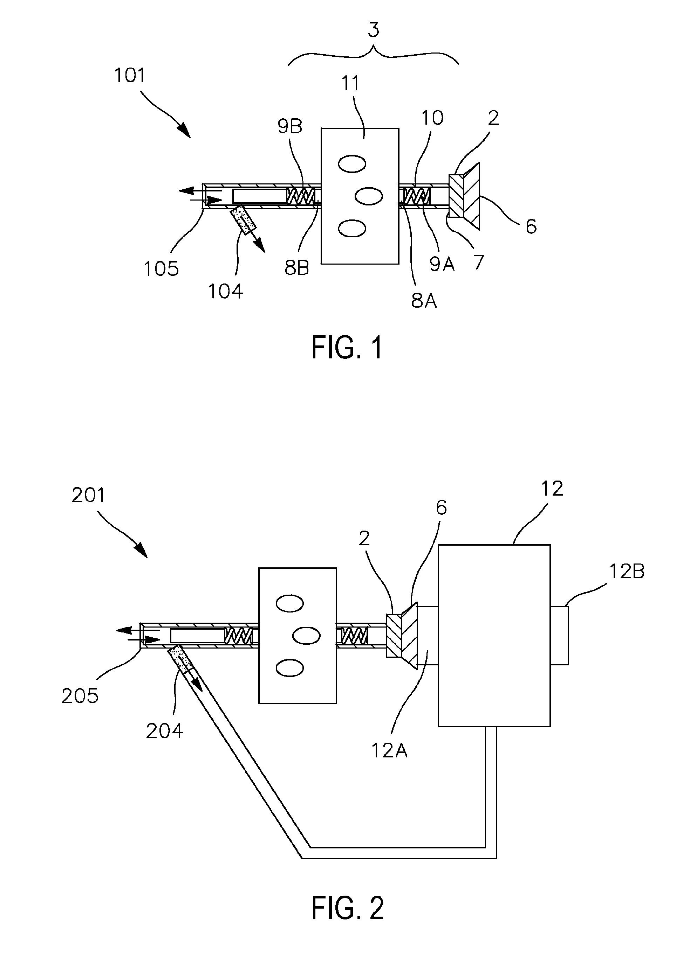

La figure 1 représente schématiquement un appareil 101 en coupe longitudinale. Cet appareil représenté schématiquement avec une forme globalement longiligne comprend successivement d'une extrémité à une autre : un moyen de prélèvement 2, un moyen de transformation 3, un moyen d'évacuation de fluide 104 qui, dans ce mode de réalisation, évacue le fluide vers l'extérieur de l'appareil et un moyen d'application 105.

Le moyen de prélèvement 2 et le moyen d'application 105 seront, en général mais non obligatoirement, prolongés par un cordon haute pression reliant le moyen de prélèvement à la source de fluide et par un cordon basse pression reliant le moyen d'application au dispositif médical.

Le moyen de prélèvement 2 peut se présenter sous la forme d'un tube (ayant une forme grossière de trapèze sur la figure 1) présentant deux extrémités. La première extrémité 6 du moyen de prélèvement 2 est adaptée à être fixée, de manière hermétique, par l'intermédiaire d'un cordon haute pression ou directement, sur une bouteille de gaz ou sur une arrivée de gaz présente le long du mur de la chambre médicalisée. La deuxième extrémité 7 du moyen de prélèvement 2 est fixée au moyen de transformation 3.

Le moyen de prélèvement 2 et le moyen de transformation 3 peuvent être d'un seul tenant.

Le moyen de transformation 3, dans le mode de réalisation de la figure 1, comprend un ensemble de pistons 8A (piston haute pression, placé à l'intérieur du compartiment) et 8B (piston basse pression, placé à l'extérieur du compartiment) et de ressorts 9A (ressort haute pression, qui est un ressort en traction) et 9B (ressort basse pression, qui est un ressort en compression) inséré dans un tube 10 placé de part et d'autre d'un compartiment 11 présentant deux entrées. Les pistons 8A et 8B, en service, viennent régulièrement ouvrir ou fermer les entrées du compartiment 11. Une extrémité du tube 10 est fixée au moyen de prélèvement 2 et l'autre extrémité est constituée, dans cette figure, du moyen d'application 105. Le moyen de transformation 3 peut être commandé électromécaniquement (commande non représentée sur cette figure 1).

Le moyen d'évacuation 104, quant à lui, est situé sur une partie du tube 10 du côté de l'extrémité constituée du moyen d'application 105. Ce moyen d'évacuation 104 peut être, par exemple, une valve monodirectionnelle.

En fonctionnement, si on utilise l'appareil 101 avec un dispositif médical à ballon, le moyen d'application 105, qui dans ce mode de réalisation est une extrémité du tube 10, est relié, par l'intermédiaire d'un cordon basse pression ou non, à un port connecteur de fluide (non représenté), lui-même relié au ballon du dispositif médical. Le moyen de prélèvement 2, quant à lui, est fixé, par l'intermédiaire d'un cordon haute pression ou non, à une source de fluide en flux continu et haute pression, par exemple une bouteille de gaz (non représentée ici). Cette source de fluide peut avoir une pression comprise entre 0,5 et 30 bars. Un fonctionnement possible est le suivant : du fluide sous haute pression, prélevé depuis la source de fluide haute pression, en flux continu, entre dans le compartiment 11 en repoussant le piston haute pression 8A vers l'intérieur du compartiment 11. Puis quand la pression à l'intérieur du compartiment 11 devient égale à celle du fluide sous haute pression, le ressort haute pression 9A , qui agit en sens inverse du flux continu de fluide, tire le piston 8A vers l'extérieur du compartiment 11 fermant le compartiment 11. De l'autre côté du compartiment 11, le piston basse pression 8B est alors repoussé vers l'extérieur du compartiment 11 par la différence de pression entre le compartiment 11 et l'intérieur du tube 10. Du fluide sort du compartiment 11 vers le tube 10 avec une pression plus faible que celle du compartiment 11. La pression dans le compartiment 11 diminuant, le ressort basse pression 9B repousse le piston basse pression 8B vers le compartiment 11. Une pulsation de fluide basse pression a ainsi été créée. Une séquence du fonctionnement a été décrite mais il faut comprendre que ce système piston-ressort-compartiment est en perpétuel équilibre et crée régulièrement des pulsations de fluide basse pression en direction du moyen d'application 105.

Dans un mode de réalisation préféré, le système piston-ressort-compartiment est couplé à une commande électromécanique. Ainsi, suivant les informations données par un opérateur à la commande électromécanique, la fréquence des pulsations et la pression de chaque pulsation peuvent être choisies en programmant ces données sur ladite commande. Par exemple, le rythme de la pulsation peut être compris entre 10 et 300 battements par minute. Ce rythme peut également être réglé sur le rythme cardiaque du patient.

Dans le cas d'un dispositif médical à ballon, le fluide circule alors de manière pulsée du moyen d'application 105, en passant par le port connecteur de fluide, jusqu'au ballon du dispositif médical pour repartir par le moyen d'évacuation. Le gonflement/dégonflement du ballon qui s'ensuit entraîne une pression pulsatile sur le dispositif médical.

Sur cette figure 1 est représenté un unique moyen d'application 105. Toutefois, l'appareil peut présenter un ou plusieurs moyens d'application. Par exemple, lorsque le dispositif médical est une combinaison, on peut envisager d'avoir un premier moyen d'application au niveau du pantalon (avec une première fréquence et pression de pulsation) et un deuxième moyen d'application au niveau de la ceinture (avec une deuxième fréquence et pression de pulsation) et un troisième moyen d'application au niveau de la veste (avec une troisième fréquence et pression de pulsation).

La figure 2 représente un mode de réalisation de l'invention qui se distingue de celui de la figure 1 par le fait que l'appareil de l'invention 201 présente une enceinte 12 et que le moyen d'évacuation 204 est relié à cette enceinte 12.

L'enceinte 12 est schématisée sous la forme d'un cube présentant deux protubérances 12A et 12B, sur deux côtés opposés, schématisant des accès à l'enceinte. Au moins l'extrémité 12B est un accès pouvant être en position fermée hermétiquement ou ouverte. La première protubérance 12A est fixée à l'extrémité 6 du moyen de prélèvement 2 et la deuxième protubérance 12B est adaptée à venir se fixer à la source de fluide en flux continu sous haute pression.

Dans ce mode de réalisation, l'appareil 201 vient se fixer à la source de fluide en flux continu sous haute pression par l'intermédiaire de l'enceinte 12, côté extrémité 12B. En fonctionnement, l'extrémité 12B est ouverte et un volume fini de fluide est prélevé de la source de fluide et enfermé dans l'enceinte 12 par fermeture hermétique de l'extrémité 12B. Puis ladite enceinte 12 est détachée de la source de fluide. Ainsi, l'appareil 201 devient autonome.

Pour parfaire l'autonomie de cet appareil 201, le moyen d'évacuation 204 est relié à ladite enceinte 12, ainsi un circuit fermé de fluide est créé (il est évident qu'un moyen compresseur devra être envisagé pour pousser le fluide arrivant du moyen d'évacuation sous basse pression dans l'enceinte 12 sous haute pression).

En fonctionnement, si on utilise l'appareil 201 de l'invention avec un dispositif médical à ballon, le système piston-ressort-compartiment délivre du fluide venant de l'enceinte 12 en flux pulsé sous basse pression. Si l'on décrit le trajet d'une pulsation de fluide, celle-ci circule au travers du moyen d'application 205 et du port connecteur de fluide pour aller gonfler le ballon du dispositif médical. Puis, cette pulsation de fluide revient vers le moyen d'évacuation 204 d'où elle est dirigée, par compression, vers l'enceinte 12 tandis qu'une nouvelle pulsation part du moyen d'application 205 vers le ballon du dispositif médical.

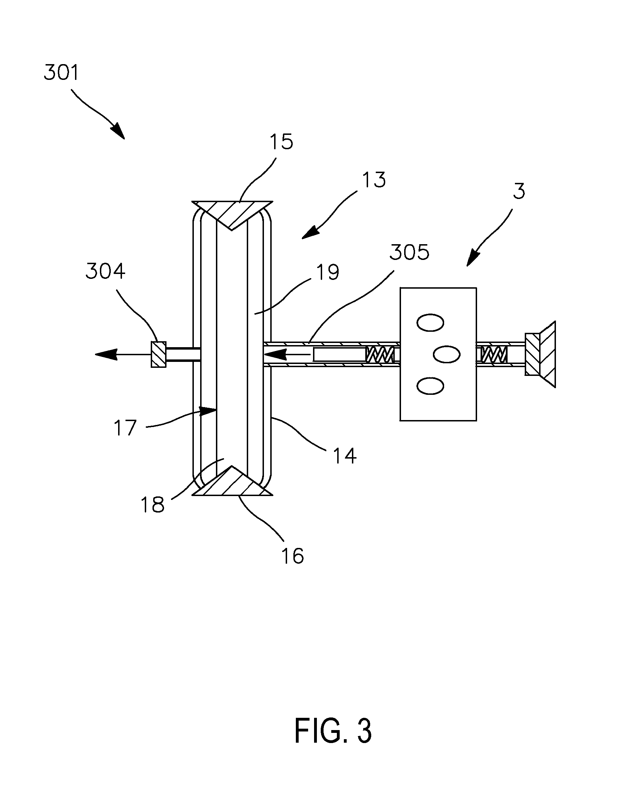

La figure 3 représente un autre mode de réalisation de l'appareil qui se distingue de celui de la figure 1 par le fait que l'appareil 301 comprend un corps creux 13 fixé audit moyen d'application 305. Ce corps creux 13, cylindrique de préférence de section circulaire, présente une paroi externe 14 et deux extrémités 15 et 16. A l'intérieur de ce corps creux 13 se trouve une membrane 17, flexible, elle-même cylindrique, reliant les deux extrémités 15 et 16, et définissant un passage traversant 18. Entre la paroi externe 14 et la membrane flexible 17 est créée une chambre creuse 19.

Ce mode de réalisation permet de créer un mouvement pulsatile sur un dispositif médical dépourvu de ballon, par exemple une tubulure, comme expliqué ci-dessous.

Du fait de la présence de ce corps creux 13, le moyen d'application 305 n'est pas relié à un port connecteur de fluide mais traverse la paroi externe 14 et aboutit dans la chambre creuse 19, le moyen d'évacuation 304 venant de la chambre creuse 19 traverse également la paroi externe 14. Par exemple, le moyen d'évacuation 304 peut être en une position diamétralement opposée à celle du moyen d'application 305.

Quand l'appareil 301 n'est pas en service, le passage traversant 18 peut être occupé par une barre et à chaque extrémité 15, 16 peuvent être placés des bouchons fermant hermétiquement le passage traversant 18. La présence de cette barre et de ces bouchons élimine toute présence de vide dans l'appareil permettant d'appliquer une pression pulsatile, lorsqu'il n'est pas en service. Ainsi aucun contaminant de quelque nature que ce soit ne vient souiller l'appareil de l'invention.

En fonctionnement, la barre ainsi que les bouchons sont retirés de l'appareil 301. A la place de la barre, on insère un dispositif médical, puis on ouvre la source de fluide (non représentée).

Le fluide en flux pulsatile sous basse pression venant du moyen de transformation 3 circule au travers du moyen d'application 305 jusque dans la chambre creuse 19. Deux fonctionnements sont alors envisagés

Dans le premier, la pulsation de fluide sous basse pression entrant dans la chambre creuse 19 vient exercer une pression à un endroit de la membrane flexible 17, pression qui se répercute sur le dispositif médical. Puis le fluide se répartit dans la chambre creuse 19 et le dispositif médical ne subit plus de pression. Le moyen d'évacuation 304 ne sert alors qu'à purger l'appareil de tout fluide. Ce fonctionnement se répète à chaque pulsation, on obtient donc un dispositif médical subissant des mouvements de compression/décompression pulsatiles.

Dans le deuxième mode de fonctionnement, la pulsation de fluide sous basse pression représente un volume de fluide supérieur à la capacité de ladite chambre creuse 19 ce qui a pour effet de comprimer la membrane flexible 17 contre le dispositif médical. Puis, le moyen d'évacuation 304 est ouvert et la chambre creuse 19 se vide de ce surplus de fluide, la membrane 17 n'étant alors plus comprimée contre les parois du dispositif médical. Ce fonctionnement se répète à chaque pulsation, on obtient donc, là encore, un dispositif médical subissant des mouvements de compression/décompression pulsatiles.

La présente invention n'est pas limitée aux formes d'exécution décrites et représentées. Par exemple, dans le moyen de transformation, un système de clapets pourrait être envisagé à la place de celui de piston-ressort. De même un mode de réalisation de l'appareil de l'invention pourrait associer enceinte 12 et corps creux 13.

## Revendications

1. Appareil d'application d'une pression pulsatile déterminée sur un dispositif médical, comprenant :
- un moyen de prélèvement (2) apte à prélever du fluide depuis une source de fluide en flux continu sous haute pression ;
- un moyen de transformation (3) apte à transformer ledit fluide en un fluide en flux pulsatile sous basse pression ;
- au moins un moyen d'application (105) pour appliquer ledit fluide, en flux pulsatile sous basse pression sur ledit dispositif médical ; et
- un moyen d'évacuation (104 ; 204) dudit fluide, et **caractérisé en ce qu'**il comprend en outre, une enceinte (12), ladite enceinte étant :
- apte à être fixée temporairement à la source de fluide en flux continu sous haute pression, pour prélever un volume fini de fluide ;
- fixée au moyen de prélèvement (2) pour permettre le prélèvement du volume fini de fluide qu'elle contient ; et
- fixée au moyen d'évacuation (204) pour créer un circuit fermé de fluide.

2. Appareil d'application d'une pression pulsatile déterminée sur un dispositif médical selon la revendication 1, **caractérisé en ce qu'**il comprend, en outre, un corps creux (13) présentant deux extrémités (15, 16) et, de la périphérie vers le centre du corps creux, une paroi externe (14), une chambre creuse (19) et une membrane flexible (17) définissant un passage traversant reliant les deux extrémités, adapté à l'insertion d'un dispositif médical, ledit moyen d'application (105) traversant ladite paroi externe, aboutissant dans la chambre creuse et étant adapté à appliquer une pression sur la membrane flexible de manière pulsée et le moyen d'évacuation partant de la chambre creuse et traversant également ladite paroi externe.

3. Appareil d'application d'une pression pulsatile déterminée sur un dispositif médical selon l'une quelconque des revendications précédentes, **caractérisé en ce que** ledit moyen de transformation (3) est un système de pistons-ressorts et de compartiment.

4. Appareil d'application d'une pression pulsatile déterminée sur un dispositif médical selon l'une quelconque des revendications précédentes, **caractérisé en ce que** le moyen de transformation (3) est commandé électro-mécaniquement.

5. Appareil d'application d'une pression pulsatile déterminée sur un dispositif médical selon l'une quelconque des revendications précédentes, **caractérisé en ce qu'**il comprend, en outre, des moyens de contrôle et d'alarme.

## Patentansprüche

1. Anwendungsgerät eines bestimmten pulsierenden Drucks auf eine medizinische Vorrichtung, umfassend :
- ein Entnahmemittel (2), das geeignet ist, Flüssigkeit von einer Flüssigkeitsquelle im kontinuierlichen Fluss unter hohem Druck zu entnehmen;
- ein Verarbeitungsmittel (3), das geeignet ist, die genannte Flüssigkeit in eine Flüssigkeit im pulsierenden Fluss unter niedrigem Druck umzuwandeln;
- wenigstens ein Anwendungsmittel (105) für die Anwendung der genannten Flüssigkeit in einem pulsierenden Fluss unter niedrigem Druck auf die genannte medizinische Vorrichtung; und
- ein Austragsmittel (104; 204) der genannten Flüssigkeit und
- **dadurch gekennzeichnet, dass**
es darüber hinaus eine Einfassung (12) umfasst, wobei die genannte Einfassung:
- geeignet ist, vorübergehend an der Quelle der Flüssigkeit in kontinuierlichem Fluss unter hohem Druck befestigt zu werden, um ein festes Flüssigkeitsvolumen zu entnehmen;
- am Entnahmemittel (2) befestigt ist, um die Entnahme des festen Flüssigkeitsvolumens, das sie enthält, zu ermöglichen; und
- anhand des Austragsmittels (204) befestigt ist, um einen geschlossenen Flüssigkeitskreislauf zu schaffen.

2. Anwendungsgerät eines bestimmten pulsierenden Drucks auf eine medizinische Vorrichtung gemäß Anspruch 1, **dadurch gekennzeichnet, dass** es darüber hinaus einen hohlen Körper (13) umfasst, der zwei Enden (15, 16) aufweist und von der Peripherie zum Zentrum des hohlen Körpers eine äußere Wand (14), eine hohle Kammer (19) und ein flexibles Organ (17) umfasst, das einen Durchgang definiert, welcher die zwei Enden verbindet, der zum Einfügen einer medizinischen Vorrichtung geeignet ist, wobei das genannte Anwendungsmittel (105) die genannte äußere Wand durchquert, in der hohlen Kammer endet und angepasst ist, um einen Druck auf das flexible Organ auf pulsierende Weise und das Austragsmittel auszuüben, das von der hohlen Kammer ausgeht und ebenfalls die genannte äußere Wand durchquert.

3. Anwendungsgerät eines bestimmten pulsierenden Drucks auf eine medizinische Vorrichtung gemäß einem der voranstehenden Ansprüche, **dadurch gekennzeichnet, dass** das genannte Verarbeitungsmittel (3) ein Kolben-Feder- und Fachsystem ist.

4. Anwendungsgerät eines bestimmten pulsierenden Drucks auf eine medizinische Vorrichtung gemäß einem der voranstehenden Ansprüche, **dadurch gekennzeichnet, dass** das Verarbeitungsmittel (3) elektrisch-mechanisch gesteuert ist.

5. Anwendungsgerät eines bestimmten pulsierenden Drucks auf eine medizinische Vorrichtung gemäß einem der voranstehenden Ansprüche, **dadurch gekennzeichnet, dass** es darüber hinaus Kontroll- und Alarmmittel umfasst.

## Claims

1. Apparatus for applying a predetermined pulsating pressure on a medical device comprising:
- drawing means (2) suitable for drawing fluid from a high-pressure continuous flow fluid source;
- conversion means (3) suitable for converting said fluid into a low-pressure pulsating flow fluid;
- at least one application means (105) for applying said fluid, in a low-pressure pulsating flow on said medical device; and
- means for discharging (104; 204) said fluid,
**characterised in that** the apparatus further comprises an enclosure (12), said enclosure being:
- suitable for being temporarily attached to the high-pressure continuous flow fluid source, to draw a finite fluid volume;
- attached to the drawing means (2) to enable the drawing of the finite fluid volume contained therein; and
- attached to the discharge means (204) to create a closed fluid circuit.

2. Apparatus for applying a predetermined pressure on a medical device according to claim 1, **characterised in that** it further comprises a hollow body (13) having two ends (15, 16) and, from the periphery to the centre of the hollow body, an outer wall (14), a hollow chamber (19) and a flexible membrane (17) defining a through passage joining both ends, suitable for inserting a medical device, said application means (105) passing through said outer wall, leading to the hollow chamber and being suitable for applying a pulsating pressure on the flexible membrane and the discharge means coming from the hollow chamber and also passing through said outer wall.

3. Apparatus for applying a predetermined pressure on a medical device according to any of the above claims, **characterised in that** said conversion means (3) is a piston-spring and compartment system.

4. Apparatus for applying a predetermined pressure on a medical device according to any of the above claims, **characterised in that** the conversion means (3) is controlled electromechanically.

5. Apparatus for applying a predetermined pressure on a medical device according to any of the above claims, **characterised in that** it further comprises monitoring and alarm means.
